Europäisches Patentamt

European Patent Office

Office européen des brevets

(11) Publication number: **0 271 364 B1**

# EUROPEAN PATENT SPECIFICATION

(45) Date of publication of patent specification: **09.09.92**   (51) Int. Cl.⁵: **A61K 35/74**

(21) Application number: **87310965.6**

(22) Date of filing: **14.12.87**

---

(54) **Preparation suitable for treating enteric disorders.**

---

(30) Priority: **12.12.86 ZA 869387**
      **20.02.87 ZA 871255**

(43) Date of publication of application:
   **15.06.88 Bulletin  88/24**

(45) Publication of the grant of the patent:
   **09.09.92 Bulletin  92/37**

(84) Designated Contracting States:
   **DE FR GB IT NL**

(56) References cited:
   EP-A- 0 006 695      EP-A- 0 137 688
   EP-A- 0 219 488      BE-A- 787 466
   DE-A- 3 509 239      FR-A- 2 508 282
   FR-M- 5 528          GB-A- 930 107
   NL-A- 7 003 420

   **TECHNIK DER WEINBEREITUNG, 1980, page
   154, Verlag Eugen Ulmer, Stuttgart, DE; R.G.
   TROOST: "Technologie des Weines"**

(73) Proprietor: **BIOREM C.C.
   17 Stuart Street
   Harrismith Orange Free State(ZA)**

(72) Inventor: **Groenewald, Pieter Willem Nicolaas
   17 Stuart Street
   Harrismith Orange Free State(ZA)**

(74) Representative: **Geering, Keith Edwin et al
   REDDIE & GROSE 16 Theobalds Road
   London WC1X 8PL(GB)**

---

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

THIS invention relates to a preparation for the treatment of enteric disorders and in particular, but not exclusively, for the treatment of diarrhoea in animals.

It is generally recognised that enteric disorders which are manifested in the affected animals suffering from diarrhoea, account for substantial financial losses arising from the death or loss in condition of the animals. Diarrhoea associated losses are particularly prevalent in young ruminants and without treatment diarrhoea generally leads to the death of the animal.

Diarrhoea in cattle and sheep may be induced by one or more pathogenic infections such as by pathogenic E. coli or Corona virus infection but it may also result from such normal activities as simply moving the animals from one habitat to another or from a change in diet. In some cases no cause for the onset of diarrhoea in young animals can be identified.

Various anti-diarrhoeal preparations are available on the market but all of these have inherent limitations and are often not indicated as general use preparations for the prophylaxis and treatment of diarrhoea in view of its cost, specificity and the potential for an acquired resistance to the products. The use of antibiotic preparations to combat infective organisms which give rise to enteric disorders have rendered certain of those organisms resistant to the more commonly used antibiotics and it has accordingly been suggested in the prior art to use probiotics instead of antibiotics. Such probiotic treatment has in general been defined as comprising the oral introduction of large numbers of beneficial micro-organisms in the digestive tract of the affected host animal but it would appear that in practice only thermophilic organisms such as Lactobacillus acidophilus had been used for this purpose.

The present invention provides a probiotic preparation comprising non-thermophilic lactic acid bacteria suitable for use in the prophylaxis and/or treatment of a wide range of enteric disorders and in particular diarrhoea-inducing disorders in animals.

According to the present invention a preparation suitable for use in treating or in the prophylaxis of enteric disorders in animals comprises two or more lactic acid bacteria selected from the phenotypes of the following species, namely: Lactobacillus bavaricus, Lactobacillus casei ssp. rhamnosus, Lactobacillus coryniformis, Lactobacillus curvatus, Lactobacillus sake, Streptococcus sp. [Enterococcus) spp. and Leuconostoc spp.

Thus, according to the invention the preparation may comprise two or more lactic acid bacteria selected from the strains Lb. sake L2622, Lb. curvatus L2623, Lb. casei [rham] L2624, Lb. curvatus L2625, Lb. sake L2626, Lb. coryniformis L2627, Lb. curvatus L2628, Lb. curvatus L2629, Lb. curvatus L2630, Lb. curvatus L2632, Lb. curvatus L2635, Lb. curvatus L2638, Lb. casei [rham] L2640, Lb. bavaricus L2641, Lb. coryniformis L2643, Lb. sake L2644, Lb. curvatus L2645, Lb. coryniformis L2546, Lb. curvatus L2647, Lb. coryniformis L2651, Lb. coryniformis L2653, Lb. curvatus L2654, Lb. curvatus L2655, Lb. sake L2656, Lb. curvatus L2657, Lb. coryniformis L2658, Lb. curvatus L2659, Streptococcus sp. L2801, Leuconostoc sp. L2806 and Leuconostoc sp. L2807, the strains being typified as set out in Example 4, part (b), incorporating Table 3.

In a preferred form of the invention the preparation comprises the combination of the thirty lactic acid bacteria strains set out above. The preparation may in addition also include one or more strains from the group comprising :
Lb. bavaricus L2648, Leuconostoc sp. L2803, and Enterococcus strains, typified as set out in Example 4 (b).

The preparation according to the invention is preferably in a dry powder form but it may also be prepared in any other form suitable for administration into the digestive tract of an animal in need of treatment. Thus the preparation may alternatively comprise a liquid composition comprising the lactic acid bacteria in a liquid carrier which is pharmaceutically acceptable and which is non-toxic to the bacteria. The liquid carrier may comprise water. The liquid carrier preferably has a nutrient dissolved therein which is suitable to sustain the bacteria. The nutrient is preferably a lactic acid bacteria specific nutrient.

The dry form of the preparation preferably comprises between $0.5 \times 10^6$ and $1 \times 10^9$ lactic acid bacteria per gram total weight.

In the preferred dry form of the invention the preparation includes, in addition to the lactic acid bacteria as aforesaid, a carrier comprising the residue of a suitable growth medium for the selected lactic acid bacteria which growth medium residue is also non-toxic to the animal intended to be treated therewith.

Thus, the carrier may comprise the lactic acid bacteria cultivation residue of any one or more of lucern meal, maize meal, wheat meal, whey, or any other non-toxic organic material capable of supporting the growth of the selected lactic acid bacteria.

In the preferred form of the preparation according to the invention the carrier comprises a lucern meal

2

residue.

The preparation according to the invention typically analyses chemically as follows by weight:

| Protein | 148,1 [23,7N] g/Kg |
|---|---|
| Fat | 98,7 |
| Ash | 47,8 |
| Fibre | 96,5 |
| Calcium | 5,3 |
| Phosphorus | 3,0 |
| Potassium | 12,3 |
| Magnesium | 2,6 |
| Sodium | 1,9 |
| Ca/P | 1,77 |

The preparation is further also preferably substantially free from Salmonella and Clostridia, at least at pathogenic levels of such organisms.

Further according to the invention there is provided a method for producing a preparation suitable for use in treating or the prophylaxis of gastric disorders in animals comprising the steps of inoculating a suitable non-toxic organic growth medium with a lactic acid bacteria inoculant comprising two or more lactic acid bacteria selected from the phenotypes of the following species, namely: Lactobacillus bavaricus, Lactobacillus casei sop. rhamnosus, Lactobacillus coryniformis, Lactobacillus curvatus, Lactobacillus sake, Streptococcus sp. [Enterococcus spp.] and Leuconostoc spp., cultivating the inoculated growth medium under conditions permitting the multiplication of the lactic acid bacteria to a preferred population, and optionally dehydrating the medium and its associated population of lactic acid bacteria under conditions which are substantially non-fatal to the lactic acid bacteria to obtain the preparation.

The inoculant preferably comprises two or more of the lactic acid bacteria of the strains Lb. sake L2622, Lb. curvatus L2623, Lb. casei [rham] L2624, Lb. curvatus L2625, Lb. sake L2626, Lb. coryniformis L2627, Lb. curvatus L2628, Lb. curvatus L2629, Lb. curvatus L2630, Lb. curvatus L2632, Lb. curvatus L2635, Lb. curvatus L2638, Lb. casei [rham] L2640, Lb. bavaricus L2641, Lb. coryniformis L2643, Lb. sake L2644, Lb. curvatus L2645, Lb. coryniformis L2546, Lb. curvatus L2647, Lb. coryniformis L2651, Lb. coryniformis L2653, Lb. curvatus L2654, Lb. curvatus L2655, Lb. sake L2656, Lb. curvatus L2657, Lb. coryniformis L2658, Lb. curvatus L2659, Streptococcus sp. L2801, Leuconostoc sp. L2806 and Leuconostoc sp. L2807, the strain typified as set out in Example 4 (b).

Alternatively, and preferably the inoculant comprises the combination of the 30 lactic acid bacteria strains set out above in combination with one or more strains from Lb. bavaricus L2648 and Leuconostoc sp. L2803, typified as set out in Example 4 (b).

The inoculum preferably includes at least 1x10$^6$ lactic acid bacteria per gram and is added to the growth medium at the rate of about 100 grams per 10 Kg. of growth medium.

The growth medium may comprise any suitable non-toxic organic medium capable of supporting the cultivation of the selected lactic acid bacteria and may, for example, comprise lucern meal, maize meal, wheat meal, whey or the like.

Where applicable the growth medium is moistened to constitute a growth medium in the form of a firm wet pulp.

The growth medium may be moistened with any suitable non-toxic liquid but preferably with water.

In one form of the invention the growth medium maybe inoculated by suspending the inoculant in a 10% m/m suspension in water at a selected pH to form a concentrated inoculant suspension, allowing the concentrated inoculum suspension to stand at room temperature for between 30 and 90 minutes, diluting the concentrated inoculant suspension in a pre-determined volume of sterile water to form an inoculated moistening suspension and mixing the moistening suspension with the growth medium to obtain an inoculated wet growth medium.

Typically the growth medium may comprise about a 1:3 [m/m] lucern meal : water mixture.

In a preferred form of the invention the inoculated growth medium is cultivated under acidic conditions. Thus the growth medium may have a starting pH of between 3.5 and 6.9. Preferably the cultivation is carried out at a starting pH of 4.5. The pH of the cultivating medium may be adjusted by means of any suitable acid and preferably by means of an organic acid such as Acetic Acid.

A suitable antimycotic preservative [food grade, non-toxic to animals] which is substantially non-toxic to the lactic acid bacteria may be incorporated in the cultivating growth medium. Thus Potassium Sorbate may be added to the cultivating growth medium in a quantity of about 0,2% by weight.

The cultivation may be allowed to proceed for a period of between 12 and 100 hours and is preferably allowed to proceed for 72 hours before drying of the cultivated product is commenced.

Where applicable the cultivated growth medium is preferably dehydrated by spreading the cultivated growth medium and passing an air flow over the spreaded product at a temperature of between 15°C and 40°C.

The dried product is preferably passed through a fine screen and homogenised to constitute the preparation in the form of a dried powder.

In an alternative form of the invention the cultivated lactic acid bacteria may be isolated from the growth medium residue to constitute a purified form of the preparation. Such isolation may be carried out by processes which are known in the trade for isolation of microorganisms.

According to a third aspect of the present invention there is provided a method for producing an inoculant suitable for use in performing the method for preparing a preparation described above, the method comprising the steps of sampling the contents of the digestive tract of a slaughtered healthy animal to obtain a representative sample of such contents, cultivating the microorganisms in such sample in a growth medium for lactic acid bacteria and dehydrating the cultivated mixture to obtain a master batch inoculant of lactic acid bacteria.

The representative sample for cultivation of lactic acid bacteria is preferably obtained from the digestive tract of a slaughtered healthy ruminant. The ruminant is preferably selected from the group comprising cattle and sheep.

During cultivation it is preferred to cultivate and thereby to enrich for two or more lactic acid bacteria identified as phenotypes of the species Lactobacillus bavaricus, Lactobacillus casei ssp. rhamnosus, Lactobacillus coryniformis, Lactobacillus curvatus, Lactobacillus sake, Streptococcus sp. [Enterococcus spp.] and Leuconostoc spp.

Preferably the cultivation and enrichment is for the lactic acid bacteria from the group Lb. sake L2622, Lb. curvatus L2623, Lb. casei [rham] L2624, Lb. curvatus L2625, Lb. sake L2626, Lb. coryniformis L2627, Lb. curvatus L2628, Lb. curvatus L2629, Lb. curvatus L2630, Lb. curvatus L2632, Lb. curvatus L2635, Lb. curvatus L2638, Lb. casei [rham] L2640, Lb. bavaricus L2641, Lb. coryniformis L2643, Lb. sake L2644, Lb. curvatus L2645, Lb. coryniformis L2546, Lb. curvatus L2647, Lb. coryniformis L2651, Lb. coryniformis coryniformis L2653, Lb. curvatus L2654, Lb. curvatus L2655, Lb. sake L2656, Lb. curvatus L2657, Lb. coryniformis L2658, Lb. curvatus L2659, Streptococcus sp.2801, Leuconostoc sp.2806 and Leuconostoc sp.2087 , the strains typified as set out in Example 4 (b).

The cultivated strains may in addition include Lb. bavaricus and different Leuconostoc spp. and Streptococcus spp, typified as set out in Example 4 (b)

The inoculant preferably includes between $0.5 \times 10^6$ and $1 \times 10^9$ lactic acid bacteria per gram.

According to a fourth aspect of the present invention it is directed at the use of lactic acid bacteria as set out above in the production of a pharmaceutical preparation for use in the treatment of enteric disorders in animals.

This aspect of the invention is further directed at the use of the lactic acid bacteria (preferably with the residue of a growth medium therefor) in the production of a preparation for use in increasing feed conversion efficiency in animals or in the prophylaxis and/or treatment of enteric disorders, particularly diarrhoea-inducing disorders in animals.

In this specification the term "aminals" is intended in include man, domesticated and wild animals, as well as members of the bird family,and references to the "pharmaceutical" field are also intended to include the veterinary field. Furthermore, the term "enteric disorders" is intended to include gastric and intestinal disorders resulting in diarrhoea or constipation, intestinal disorders such as tape worm infection, as well as disorders affecting other organs, for example, disorders such as pulpy kidney. In the case of disorder manifesting itself in diarrhoea, the disorder may be as a result of Salmonella, Shigella, E. coli, Corona virus, coccidiosis, or any other infection.

The strain numbers of the various Lactobacilli strains referred to in this specification are the control numbers under which those strains are deposited in the Lactobacilli culture collection of the University of Pretoria, Republic of South Africa. The strains so identified are available from that source. The strains in question are also available from a natural source namely from the digestive tract of a ruminant and such strains can be typified with reference to their in vitro antagonism against the thirteen test strains of enteropathogenic bacteria set out in Table 3 in Example 2 below. Those test strains are available from the culture collection of the University of Pretoria or from the South African Institute for Veterinary Sciences at Onderstepoort, Republic of South Africa.

Without limiting the scope of the present invention, the invention will now be illustrated with reference to the accompanying examples.

EXAMPLE 1

Preparation of Inoculant

From the digestive tract of a freshly slaughtered well fed healthy ox a sample was taken from the contents of the rumen and other locations along the digestive tract to obtain a representative combined sample of the contents of the entire digestive tract.

The sample so obtained and containing lactic acid bacteria was cultivated for 72 hours at 27°C on lucern meal which was moistened with sterile water which was pH adjusted to a pH of 5.5. The culture was dried under airflow and the dried residue was used to inoculate a new batch of lucern meal. This process was repeated several times to obtain a symbiotic stable climax or dominant population of the lactic acid bacteria. The resultant final residue product was used as a master inoculant in the production of the preparations described below.

EXAMPLE 2

Production of the Preparation RB

50 Kg of finely sifted dry lucern meal was weighed into a fermentation vessel. About 150 litres of tap water was pH adjusted with the aid of Acetic Acid to a pH of 4,5. A fungus inhibiting effective amount [about 0,2% by weight] of Potassium Sorbate was added to the water.

About 100 to 200 grams of the master inoculant prepared as described in Example 1 above was then mixed with the pH adjusted water and the inoculated water was added to the weighed out lucern meal to create a pulp.

The pulp was allowed to stand for 72 hours at ambient temperature to allow the bacteria to grow. During growth gases escaped from the broth. Exclusion of air was brought about by overlaying the vessel with plastic bags filled with water; alternatively an anaerobic atmosphere may be created in a hermetically sealed fermentation vessel.

After 72 hours the broth was spread over a drying table and dried in a wind tunnel at about 40°C.

The dried product was passed through a screen to obtain a fine powder which was homogenised. The preparation so obtained which was coded RB.

The dried product was yellowish brown to olive green yellow in colour and its chemical analysis was as follows:

<u>g/Kg</u>

| | |
|---|---|
| **Protein** | **148,1 [23,7N]** |
| **Fat** | **98,7** |

| | |
|---|---|
| Ash | 47,8 |
| Fibre | 96,5 |
| Calcium | 5,3 |
| Phosphorus | 3,0 |
| Potassium | 12,3 |
| Magnesium | 2,6 |
| Sodium | 1,9 |
| Ca/P | 1,77 |

An alternative and purer form of the preparation was prepared in the same manner except that the lucern meal was steam sterilized for 2 hours before being inoculated with an alternative inoculant which alternative inoculant was prepared by cultivating the inoculant of Example 1 by the pour plate method on MRS and Rogosa agar, as is known in the trade, and collecting and combining the lactic acid bacteria which formed colonies on those media. The isolates obtained in this manner served as alternative inoculant for a production batch prepared in the manner as described above. The product obtained in this manner was coded RB-P and contained an undetermined part of the full spectrum of the dominant lactic acid bacteria population referred to above.

EXAMPLE 3

Quantitative Analysis and Stability of RB

Quantitative analysis of the lactic acid bacteria population in the RB product was carried out by making a 10% m/m suspension of RB in sterile water of pH 5.5 by adjustment with Acetic Acid. The suspension was left at room temperature after which a dilution was made with the same pH adjusted water.

Rogosa-agar was made up according to suppliers' directions, heated and placed in Petri-dishes onto which the inoculant was then incubated for 4 days at 28°C and at a humidity of 70%. All visible colonies were taken to be lactic acid bacteria.

Samples of RB prepared as described in Example 2 were stored in paper bags at 25°C and were subjected to weekly analyses over a period of 2 months. A final analysis was conducted after a further 6 weeks.

Standard Microbiological analyses were conducted as follows:
- serial dilutions were prepared by homogenising 20g samples of RB in 180 ml quarter strength Ringer buffer [sterile] for 2 minutes in a Stomacher.
- Further dilutions were made from this 1:10 dilution in 9 ml dilution blanks.
- TOTAL AEROBIC MICROBE POPULATION ["cultivated microbes"] were determined on Standard I - agar [Merck]; incubation:aerob. 2-3 days/30°C.
- TOTAL LACTIC ACID BACTERIA COUNT :
  - MRS-agar with 0.1% K-sorbate pH 5.7; anaerobic incubation at 30°C/3 days
  - Rogosa-agar [Merck] [pH 5.4] : anaerobic incubation at 30°C/4-5 days.
- FUNGI : direct on St. I-agar based on distinguished colony-type.

The results of these determinations are set out in Table 1.

6

TABLE I

| Storage Time Weeks | Viable Counts per gram of RB | | | |
|---|---|---|---|---|
| | Total Population | Lactobaccili | | Fungi |
| | | MRS | Rogosa | |
| 0 | $5,9 \times 10^8$ | $5,8 \times 10^8$ | $1,5 \times 10^8$ | $9,5 \times 10^6$ |
| 1 | $3,0 \times 10^9$ | $5,9 \times 10^8$ | $9,5 \times 10^8$ | $2,0 \times 10^7$ |
| 2 | $3,1 \times 10^9$ | $4,2 \times 10^8$ | $8,2 \times 10^8$ | $1,0 \times 10^7$ |
| 3 | $3,2 \times 10^9$ | $4,0 \times 10^8$ | $3,6 \times 10^8$ | $7,0 \times 10^6$ |
| 4 | $1,5 \times 10^9$ | $1,7 \times 10^8$ | $1,7 \times 10^8$ | $7,1 \times 10^6$ |
| 5 | $9,8 \times 10^8$ | $1,5 \times 10^7$ | $1,1 \times 10^7$ | $7,0 \times 10^6$ |
| 6 | $9,7 \times 10^8$ | $1,4 \times 10^8$ | $1,1 \times 10^8$ | $8,0 \times 10^6$ |
| 7 | $9,7 \times 10^8$ | $1,4 \times 10^8$ | $1,2 \times 10^8$ | $1,1 \times 10^7$ |
| 8 | $1,4 \times 10^8$ | $1,6 \times 10^8$ | $4,5 \times 10^7$ | $2,0 \times 10^7$ |
| 18 | $3,2 \times 10^8$ | $1,3 \times 10^8$ | $4,2 \times 10^7$ | $1,0 \times 10^7$ |

It can be seen that there has been no major reduction in the number of microbes over the period and that the lactic acid bacteria represent between 50% and 90% of the total population. It must also be borne in mind that all the lactic acid bacteria did not necessarily grow on the selective media and that their population could in fact be higher.

On the basis of extrapolated test results it is expected that RB would have a lactic acid bacteria population of at least $1 \times 10^6$/g after 6 months' storage.

EXAMPLE 4

Qualitative Analysis

Representative isolates were made from the plate counts referred to in Example 3 over the first 3-4 weeks. Orientation investigations were conducted [by microscope and catalase-tests] after initial purification to confirm that all isolates were lactic acid bacteria.

[a] For phenotype characterisation the following tests were conducted:
- fermentation potential in respect of 29 different sugars,
- hydrolysis of arginine,
- configuration of formed lactic acid,
- presence of diaminopimelic Acid in the cellular wall,
- slime formation from sucrose,
- growth in 4.5% and 10% NaCl
- growth at 4°C, 15°C, 37°C and 45°C,
- reaction in litmusmilk.

The results are summarized in Table 2.

TABLE 2

| Phenotype Characterization of lactic acid bacteria in RB | |
|---|---|
| | Number of strains |
| Lactobacillus bavaricus | 2 |
| Lactobacillus casei ssp. rhamnosus | 2 |
| Lactobacillus coryniformis | 7 |
| Lactobacillus curvatus | 14 |
| Lactobacillus sake | 4 |
| Streptococcus sp. | 1 |
| Leuconostoc spp. | 1 |

[b] The in vitro antagonism of the thirty-two isolated lactic acid bacteria strains against thirteen entero-pathogenic test strains were determined to typify the thirty-two strains isolated from the RB product. The various isolates of lactic acid bacteria were grown on MRS agar and discs were cut from the cultivated medium. The entero-pathogenic test strains were inoculated onto Standard I-agar by the pour plate method and the discs of the cultivated lactic acid bacteria were placed on the inoculated medium. After incubation for 1 and 2 days at 30°C the diameters of the resultant clear inhibition zones were measured. The results are summarized below in Table 3 in which the extent of inhibition is indicated as follows:

-       inhibition zone smaller than 0,5 cm

+       inhibition zone larger than 0,5 cm but smaller than 1,0 cm

+ +     inhibition zone larger than 1.0 cm

TABLE 3

| LACTOBACILLUS ISOLATES | | TEST STRAINS | | | | | | | | | | | | |
|---|---|---|---|---|---|---|---|---|---|---|---|---|---|---|
| SPECIES / STRAIN NO. | | S. java (UP) | S. typhimurium (UP) | Strep. Faecium (UP) | S. typhi 2005 | S. typhi 1839 | S. anatum 840 | S. loma-linda 2463 | E. coli 8082 | E. coli 7917 | E. coli 7821 | E. coli 8025 | E. coli 8386 | E. coli 7734 |
| Lb. sake | L2622 | ++ | ++ | - | ++ | ++ | ++ | ++ | ++ | + | - | + | + | ++ |
| Lb. curvatus | L2623 | + | ++ | - | ++ | ++ | ++ | ++ | ++ | - | - | ++ | - | - |
| Lb. casei (rham) | L2624 | ++ | ++ | - | ++ | ++ | ++ | - | ++ | + | - | ++ | + | - |
| Lb. curvatus | L2625 | ++ | ++ | - | ++ | ++ | ++ | ++ | ++ | - | ++ | + | - | - |
| Lb. sake | L2626 | - | - | - | - | - | - | + | - | - | - | + | - | - |
| Lb. coryniformis | L2627 | + | + | - | + | + | + | + | + | - | - | - | + | + |
| Lb. curvatus | L2628 | ++ | ++ | - | ++ | ++ | ++ | ++ | ++ | + | - | ++ | - | + |
| Lb. curvatus | L2629 | - | ++ | - | - | - | - | - | + | + | - | - | - | - |
| Lb. curvatus | L2630 | - | ++ | - | - | - | - | - | - | - | - | - | - | - |
| Lb. curvatus | L2632 | ++ | ++ | - | ++ | + | ++ | ++ | ++ | - | ++ | ++ | - | + |
| Lb. curvatus | L2635 | ++ | + | ++ | ++ | ++ | ++ | - | ++ | - | - | ++ | - | - |
| Lb. curvatus | L2638 | - | - | ++ | - | - | - | - | - | + | + | + | - | - |
| Lb. casei (rham) | L2640 | - | - | - | - | - | - | + | - | - | - | - | - | - |
| Lb. bavaricus | L2641 | - | - | + | - | - | - | - | - | - | - | - | - | - |
| Lb. coryniformis | L2643 | + | ++ | - | ++ | + | + | ++ | + | + | - | - | + | + |
| Lb. sake | L2644 | - | ++ | + | ++ | ++ | ++ | ++ | ++ | - | - | + | ++ | - |
| Lb. curvatus | L2645 | - | + | ++ | + | + | + | - | + | + | + | - | - | + |
| Lb. coryniformis | L2546 | + | ++ | ++ | ++ | ++ | ++ | ++ | - | - | + | ++ | - | - |
| Lb. curvatus | L2647 | + | ++ | ++ | ++ | ++ | ++ | - | + | - | + | ++ | - | - |
| Lb. bavaricus | L2648 | - | - | - | - | - | - | - | - | - | - | - | - | - |
| Lb. coryniformis | L2651 | ++ | ++ | ++ | ++ | ++ | + | + | + | + | - | ++ | + | - |
| Lb. coryniformis | L2653 | - | ++ | - | + | + | + | ++ | + | + | - | - | - | - |
| Lb. curvatus | L2654 | - | + | - | + | + | + | - | + | + | - | - | - | + |
| Lb. curvatus | L2655 | + | + | - | + | + | + | - | + | + | - | - | - | - |
| Lb. sake | L2656 | ++ | ++ | ++ | ++ | + | ++ | ++ | ++ | + | - | + | - | ++ |
| Lb. curvatus | L2657 | + | ++ | ++ | ++ | - | ++ | + | ++ | - | + | ++ | - | - |
| Lb. coryniformis | L2658 | + | + | ++ | ++ | + | ++ | + | ++ | - | - | ++ | - | - |
| Lb. curvatus | L2659 | - | + | - | - | - | - | - | - | - | - | - | - | - |
| Streptococcus sp. | L2801 | - | - | - | - | - | - | - | - | - | + | - | - | - |
| Leuconostoc sp. | L2803 | - | - | - | - | - | - | - | - | - | - | - | - | - |
| Leuconostoc sp. | L2806 | - | - | - | - | - | - | - | - | - | - | - | + | - |
| Leuconostoc sp. | L2807 | - | - | - | - | - | - | - | - | + | - | - | - | - |

Extent of inhibition

-       inhibition zone 0.5cm

+       inhibition zone 0.5 cm but 1.0 cm

+ +      inhibition zone 1.0 cm

EXAMPLE 5

Toxicity

[a] Overdosage
The recommended dosage of RB for young calves is 4g per feeding twice daily.
No change in condition, general state of health of body weight of young calves were observed in comparison with an untreated control group of young calves when
[i] 16g were administered twice a day for 6 weeks, i.e. 4 times recommended dosage, or
[ii] 20 times recommended dosage was administered twice a day for 5 days, or
[iii] 125 times recommended dosage was administered over two days.
[b] Since the preparation is intended for administration to young animals and not adult animals, the determination of teratogenic of mutagenic effects were considered unnecessary.
[c] Residues
Since the product composition is substantially equivalent to part of the content of the digestive tract, no metabolites could be determined in the muscles of treated animals.

EXAMPLE 6

Prophylactic Treatment against Diarrhoea

On a dairy farm with a stud of about 60 Frisian cows the survival history of hand raised calves over 3 years was as follows:

| Year | Calves born | Fatalities | Survivors |
|------|-------------|------------|-----------|
| 1 | 37 | 31 | 6 |
| 2 | 49 | 38 | 11 |
| 3 | 49 | 38 | 11 |

Accordingly the average survival rate was 20.3% and most of the calves died after serious diarrhoea.
In the fourth year all calves were treated with RB by suspending 4g of RB powder in their milk at each feeding. No anti-bacterial treatment was administered during this period. The calves were allowed with the mothers for two days after birth and then separated into camps on kikuyu where roughage, calf growth meal and fresh water was constantly available. The calves were inoculated against calf paratyphus and pasteurella at 1 and 4 weeks and dosed with MULTISPEC R [a product marketed by Janssen Pharmaceuticals] in the first and fourth months depending on condition.
The survival rate was as follows:

| Year | Calves born | Fatalities | Survivors |
|------|-------------|------------|-----------|
| 4 | 54 | 5 | 49 |

Accordingly a survival rate of 90.7% was achieved and those deaths that did occur were not diarrhoea-linked.

EXAMPLE 7

Curative Treatment of Diarrhoea of Unknown Cause

Nine Frisian calves of age between 3 and 5 days and all suffering of heavy diarrhoea were placed in separate pens and two calves were taken as controls and received no treatment. The other seven were given 8g of RB-P in $\frac{1}{2}$ litre milk per feeding session for the first three days from commencement of the test and in all 7 cases the diarrhoea was stopped except in two cases were a light diarrhoea was still present. On the fourth day no treatment was given. On the fifth day the two calves showing signs of light diarrhoea were again given 8g of RB-P in ½ litre of milk while the others were given 4g of RB-P in ½ litre of milk per feeding session.

Treatment at the rate of 4g of RB-P was continued for all seven up to weaning at six weeks except for one of the treated calves which died of pneumonia on day 27.

The two untreated calves both showed continued deterioration and both died on the third day after the test commenced after suffering from very heavy diarrhoea.

In all cases the calves had free access to growth meal, roughage and water.

The cause of the diarrhoea was not established in this test.

EXAMPLE 8

Treatment of E. coli rough induced diarrhoea

Two Frisian calves respectively 18 and 22 days of age and both suffering from very heavy diarrhoea were treated by the administration of 8g RB-P twice daily.

The faeces of both calves were analysed before treatment by the S.A. Research Institute for Veterinary Sciences and found to contain E.coli Rough. In one case the faeces was whitish in colour and in the other it was bloody.

After only twelve hours of treatment the diarrhoea started subsiding and it stopped completely after 36 hours.

Treatment at 2 x 8g RB-P per day was continued for 7 days and then changed to 2 x 4g RB-P per day for a further 14 days.

Both calves recovered completely.

EXAMPLE 9

Treatment of a known pathogenic E. coli induced diarrhoea

Three healthy 2 day old calves were placed in separate pens. For the first three days each calf was given 1 litre colostrum twice daily and 1 litre lukewarm water daily. From the sixth day the colostrum was replaced by milk surrogate.

From the start of the test one calf was given 4g RB per feeding session and a second calf was given 4g RB-P per feeding session. The third calf was left untreated.

At the age of seven days all three calves were infected with a dose of pathogenic E. coli 09:K30,99 which had been prepared by the Research Institute for Veterinary Sciences.

The untreated calf developed diarrhoea on the second day after infection which increased over the next day and the calf died on the following day.

The calf treated with RB showed no signs of diarrhoea and was weaned at the age of 43 days.

The calf treated with RB-P developed a light diarrhoea and the treatment dosage was increased to 8g RB-P per feeding session for 3 days. The diarrhoea cleared after one day and the treatment was reduced to the normal dose until weaning at the age of 43 days.

EXAMPLE 10

Treatment of Corona-virus induced diarrhoea

On a Jersey stud dairy farm with animals of very high quality the experience had been that calves which spent two days with their mothers developed a whitish diarrhoea which turns reddish after being separated. Although relatively few calves died as the condition normally resolved itself within three days, it occurrence was considered bad for the image of the stud.

The Research Institute for Veterinary Sciences isolated Corona virus from the faeces of affected calves and several attempts to improve the situation had been unsuccessful.

After the calves were placed under treatment comprising the administration of 4g RB per feeding session, the dairrhoea stopped but when the treatment was interrupted for four days, the typical diarrhoea reappeared.

EXAMPLE 11

Treatment of Pulpy Kidney

It has been observed that transportation of sheep or a change in the feedstuff of sheep often gives rise to an increased incidence of fatalities as a result of pulpy kidney in such sheep.

[a] To evaluate the effect of RB on sheep exposed to conditions which increase the risk of pulpy kidney incidence, a flock of 50 Merino sheep was bought in the Karroo and transported to a feedlot in the Transvaal where feedstuff adaptation has often in the past resulted in death due to pulpy kidney.

Half the flock received no treatment as a control group while the other 25 were given 4 grams of RB per day by placing the dry powder on the tongue for 3 days after arrival.

The control group suffered from a significant mass loss and three of the 25 died as a result of pulpy kidney.

The treated group did not undergo any significant loss in mass and no deaths occurred in this group.

[b] In vitro tests on the organism causing pulpy kidney showed that such organism was substantially eliminated by means of the RB product.

EXAMPLE 12

Coccidiosis

Preliminary indications obtained from tests done on lambs which suffered from typical coccidiosis symptoms indicate an activity of RB against that form of infection. These tests are being continued.

EXAMPLE 13

Tape Worm Control

Work to evaluate the control afforded by RB against tape worm infestation has been promising enough to warrant further investigation.

EXAMPLE 14

Salmonella

A flock of feed-lot sheep suffering from Salmonella induced diarrhoea was divided into four groups. Two groups were treated with registered chemotherapeutic compositions indicated for Salmonella infection, a third group was used as an untreated control and the fourth group was treated with RB.

All the RB treated animals recovered while te untreated and chemotherapy treated control groups all suffered casualties ranging from 30 to 50% of the infected animals.

EXAMPLE 15

Feed Conversion Efficiency

Two groups of merino lambs of approximately the same starting mass [28 Kg average] were given the same rations over a period of 63 days before slaughter. A test group was additionally also given 10 millilitres of liquid RB product prepared as described in Example 16 below at the start of the test while the control group received no RB.

The average daily mass gain [live mass] of the test group was 10,8% higher than that of the control group which represented a feed conversion efficiency of 5,9% better for the control group. The final carcass mass gain of the treated group was about 8,6% better than that of the untreated group while the feed conversion to carcass mass was 10,7% better in the treated group compared to the untreated group. The grading of the meat of the treated group was also higher. These factors combined to give a 25,9% better return on cost for the treated group relative to the untreated group.

The same experiment was conducted on adult sheep [average starting mass 35 Kg] and in this case the final return on cost for the treated group was 23,8% better than for the untreated group.

EXAMPLE 16

Preparation of Liquid Composition and Kit for use therein

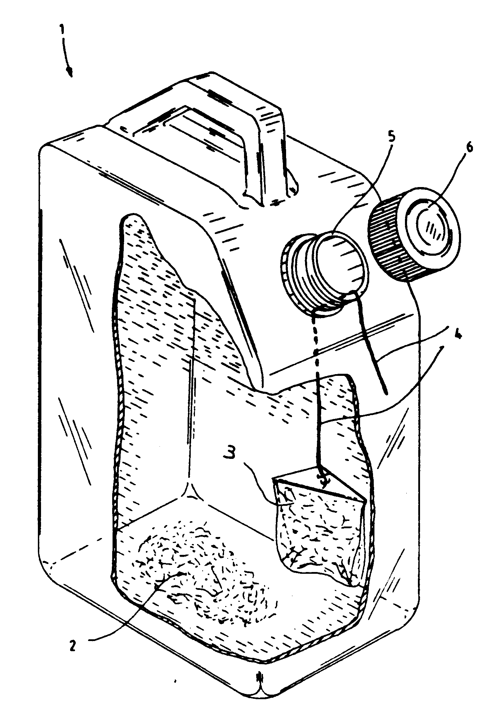

A kit for use in producing a liquid composition is illustrated in Figure 1 which is a partly broken away perspective view of the container forming part of the kit to illustrate the other elements. The kit typically comprises a 1 litre container 1 made from a polymeric material and into which is placed a nutrient mixture 2 comprising:

| | |
|---|---|
| Peptone | 4 grams |
| Yeast Extract | 5 grams |
| Glucose | 8 grams |
| Triammonium Citrate | 3 grams |
| Sodium Acetate | 5 grams |
| Magnesium Sulphate | 0,1 grams |
| Manganese Sulphate | 0,05 grams |
| Di Potassium Phosphate | 2 grams |
| Potassium Sorbate | 1 gram |
| Malic Acid | to bring pH of mixture in water to about 5,8 |

and an inoculating lactic acid bacteria mixture in a holder as described below.

The holder is in the form of a permeable paper bag 3 of the kind conventionally used as a tea or coffee bag into which a 100 gram quantity of the RB product prepared as described in Example 2 above was placed.

The bag 3 was fitted with a string 4 by means of which it may be suspended in the container 1 and it may be held in place by nipping the string 4 between the pouring spout 5 and cap 6 of the container.

In use the container containing the nutrient was simply filled with water and shaken to dissolve the nutrient. The bag 3 was then introduced into the nutrient solution and suspended in place by nipping the string 4 between the spout 5 and cap 6 which was loosely fitted on the spout to allow the escape of gases from the container.

The container was allowed to stand for about 24 hours at ambient temperature to allow cultivation of the lactic acid bacteria. The water mixture became milky during this period due to the presence of the bacteria.

The inoculating bag was removed and the liquid composition was administered orally by means of a syringe-type dosing gun to animals in need of treatment. The product so obtained is referred to as liquid RB in Example 15 above. 5 to 10 ml dosages of this product may be administered to animals depending on size. The efficiency of the product so prepared was comparable with that of the dry RB product described above.

Many variations of the invention can be made without departing from the spirit of the invention. Successful treatments have also been achieved on hand raised lambs [at 1g per single feeding per day] as well as on man but tests in confirmation of these results are still being conducted.

**Claims**

1. A preparation suitable for use in the treatment or prophylaxis of enteric disorders in animals comprising two or more lactic acid bacteria selected from phenotypes of the following species, namely: Lactobacillus bavaricus, Lactobacillus casei ssp. rhamnosus, Lactobacillus coryniformis, Lactobacillus curvatus, Lactobacillus sake, Streptococcus sp. [Enterococcus spp.] and Leuconostoc spp.

2. A preparation according to claim 1 which comprises lactic acid bacteria of two or more (preferably all) of the strains Lb. sake L2622, Lb. curvatus L2623, Lb. casei [rham] L2624, Lb. curvatus L2625, Lb. sake L2626, Lb. coryniformis L2627, Lb. curvatus L2628, Lb. curvatus L2629, Lb. curvatus L2630, Lb. curvatus L2632, Lb. curvatus L2635, Lb. curvatus L2638, Lb. casei [rham] L2640, Lb. bavaricus L2641, Lb. coryniformis L2643, Lb. sake L2644, Lb. curvatus L2645, Lb. coryniformis L2546, Lb. curvatus L2647, Lb. coryniformis L2651, Lb. coryniformis L2653, Lb. curvatus L2654, Lb. curvatus L2655, Lb. sake L2656, Lb. curvatus L2657, Lb. coryniformis L2658, Lb. curvatus L2659, Streptococcus sp. L2801, Leuconostoc sp. L2806 and Leuconostoc sp. L2807, the strains being typified as set out in part (b) of Example 4, incorporating Table 3.

3. A preparation according to claim 2 which in addition includes one or more strains selected from Lb. bavaricus L2648 Leuconostoc sp. L2803, typified as set out in Example 4 (b), incorporating Table 3.

4. A preparation according to any one of claims 1 to 3 which is (a) in the form of a dry powder which preferably includes a pharmaceutically acceptable carrier in the form of the residue of a suitable growth medium for the lactic acid bacteria (e.g. a growth medium selected from lucern meal, maize meal, wheat meal and whey) or (b) in the form of a liquid composition which is preferably an aqueous composition containing a water soluble lactic acid bacteria specific nutrient.

5. A method for producing a preparation suitable for use in the treatment or prophylaxis of enteric disorders in animals comprising the steps of inoculating a suitable non-toxic organic growth medium with a lactic acid bacteria inoculant comprising two or more lactic acid bacteria selected from the phenotypes of the following species, namely: Lactobacillus bavaricus, Lactobacillus casei ssp. rhamnosus, Lactobacillus coryniformis, Lactobacillus curvatus, Lactobacillus sake, Streptococcus sp. - [Enterococcus spp.] and Leuconostoc spp., cultivating the inoculated growth medium under conditions permitting the multiplication of the lactic acid bacteria to a preferred population, and optionally dehydrating the medium and its associated population of lactic acid bacteria under conditions which are substantially non-fatal to the lactic acid bacteria to obtain the preparation.

6. A method according to claim 5 in which the inocculant comprises a mixture of lactic acid bacteria of two or more (preferably all) of the strains Lb. sake L2622, Lb. curvatus L2623, Lb. casei [rham] L2624, Lb. curvatus L2625, Lb. sake L2626, Lb. coryniformis L2627, Lb. curvatus L2628, Lb. curvatus L2629, Lb. curvatus L2630, Lb. curvatus L2632, Lb. curvatus L2635, Lb. curvatus L2638, Lb. casei [rham] L2640, Lb. bavaricus L2641, Lb. coryniformis L2643, Lb. sake L2644, Lb. curvatus L2645, Lb. coryniformis L2546, Lb. curvatus L2647, Lb. coryniformis L2651, Lb. coryniformis L2653, Lb. curvatus L2654, Lb. curvatus L2655, Lb. sake L2656, Lb. curvatus L2657, Lb. coryniformis L2658, Lb. curvatus L2659, Streptococcus sp. L2801, Leuconostoc sp. L2806 and Leuconostoc sp. L2807, the strains typified as set out in Example 4 (b) incorporating Table 3.

7. A method according to claim 6 in which the inoculant also includes at least one of the strains Lb. bavaricus L2648 and Leuconostoc sp. L2803, typified as set out in Example 4 (b), incorporating Table 3.

8. A method according to claim 5,6 or 7 wherein the growth medium is selected from lucern meal, maize meal, wheat meal and whey, preferably a slurry of lucern meal in water in a weight ratio of about 1:3 with the water preferably adjusted to a pH of between 3.5 and 6.9 (e.g. about 4.5).

9. A method according to any one of claims 5 to 8 wherein the growth medium includes an anti-mycotic preservative which is non-toxic to lactic acid bacteria, preferably potassium sorbate.

10. A method according to any of claims 5 to 9 in which the cultivated growth medium is dehydrated by

14

spreading the cultivated growth medium and passing an air flow over the spread product at a temperature of between 15°C and 40°C, the dried product optionally being passed through a fine screen and homogenised to obtain the preparation in the form of a fine dried powder.

11. A method of producing an inoculant suitable for use in a method according to any of claims 5 to 10 comprising the steps of sampling the contents of the digestive tract of a slaughtered healthy animal to obtain a representative sample of such contents, cultivating the microorganisms in such sample in a suitable growth medium for lactic acid bacteria and dehyrating the cultivated mixture to obtain a master batch inoculant of lactic acid bacteria, the animal preferably being a ruminant, preferably selected from cattle and sheep.

12. A method for producing a liquid preparation suitable for use in the treatment or prophylaxis of intestinal disorders in animals comprising the steps of providing an inoculant produced by a method according to any of claims 5 to 10 in a permeable holder therefor (preferably a permeable paper bag) locating the holder in a liquid growth medium comprising water and a solubilized nutrient for the lactic acid bacteria, allowing the lactic acid bacteria to cultivate and permeate through the holder into the medium and removing the holder from the liquid preparation so obtained, the nutrient preferably comprising a combination of the following products in the mass ratio substantially as specified:

| | |
|---|---|
| Peptone | 4 parts |
| Yeast Extract | 5 parts |
| Glucose | 8 parts |
| Triammonium Citrate | 3 parts |
| Sodium Acetate | 5 parts |
| Magnesium Sulphate | 0,1 parts |
| Manganese Sulphate | 0,05 parts |
| Di Potassium Phosphate | 2 parts |
| Potassium Sorbate | 1 part |
| Malic Acid | to bring pH of aqueous solution of the nutrients to about 5.8. |

13. The use of two or more lactic acid bacteria selected from the phenotypes of the following species, namely: Lactobacillus bavaricus, Lactobacillus casei ssp. rhamnosus, Lactobacillus coryniformis, Lactobacillus curvatus, Lactobacillus sake, Streptococcus sp. [Enterococcus spp.] and Leuconostoc spp. in the manufacture of a pharmaceutical preparation for use in increasing feed conversion efficiency in animals or in the treament or prophylaxis of enteric disorders in animals.

14. A kit for use in producing a liquid preparation suitable for use in the treatment or prophylaxis of enteric disorders in animals comprising a container suitable in use to be filled with water, the container containing a dry water soluble nutrient for lactic acid bacteria and a removable holder of material permeable to lactic acid bacteria and containing a bacteria mixture as defined in any of claims 1 to 3, the nutrient preferably comprising a mixture of the following products substantially in the mass ratio indicated:

| | |
|---|---|
| Peptone | 4 parts |
| Yeast Extract | 5 parts |
| Glucose | 8 parts |
| Triammonium Citrate | 3 parts |
| Sodium Acetate | 5 parts |
| Magnesium Sulphate | 0,1 parts |
| Manganese Sulphate | 0,05 parts |
| Di Potassium Sulphate | 2 parts |
| Potassium Sorbate | 1 part |
| Malic Acid | to bring the pH of a mixture of the products in water to 5.8. |

**15.** A preparation according to claim 1, containing lactic acid bacteria from all of said species.

**Patentansprüche**

1. Zubereitung, geeignet zur Verwendung bei der Behandlung oder Prophylaxe von Darmkrankheiten bei Tieren, umfassend zwei oder mehrere Milchsäurebakterien, ausgewählt aus Phänotypen der folgenden Spezies, nämlich:
Lactobacillus bavaricus, Lactobacillus casei ssp. rhamnosus, Lactobacillus coryniformis, Lactobacillus curvatus, Lactobacillus sake, Streptococcus sp. (Enterococcus spp.) und Leuconostoc spp.

2. Zubereitung nach Anspruch 1, die Milchsäurebakterien von zwei oder mehreren (vorzugsweise sämtliche) Stämme von Lb. sake L2622, Lb. curvatus L2623, Lb. casei (rham) L2624, Lb. curvatus L2625, Lb. sake L2626, Lb. coryniformis L2627, Lb. curvatus L2628, Lb. curvatus L2629, Lb. curvatus L2630, Lb. curvatus L2632, Lb. curvatus L2635, Lb. curvatus L2638, Lb. casei (rham) L2640, Lb. bavaricus L2641, Lb. coryniformis L2643, Lb. sake L2644, Lb. curvatus L2645, Lb. coryniformis L2546, Lb. curvatus L2647, Lb. coryniformis L2651, Lb. coryniformis L2653, Lb. curvatus L2654, Lb. curvatus L2655, Lb. sake L2656, Lb. curvatus L2657, Lb. coryniformis L2658, Lb. curvatus L2659, Streptococcus sp. L2801, Leuconostoc sp. L2806, und Leuconostoc sp. L2807 umfaßt, wobei die Stämme wie im Teil (b) des Beispiels 4 einschließlich Tabelle 3 angegeben typisch sind.

3. Zubereitung nach Anspruch 2,
die zusätzlich eine oder mehrere Stämme umfaßt, die aus Lb. bavaricus L2648 Leuconostoc sp. L2803 ausgewählt werden, wie sie im Beispiel 4 (b), einschließlich Tabelle 3, dargestellt sind.

4. Zubereitung nach irgendeinem der Ansprüche 1 - 3,
die (a) in Form eines Trockenpulvers vorliegt, das vorzugsweise einen pharmazeutisch annehmbaren Träger in Form des Restes eines geeigneten Zuchtmittels für Milchsäurebakterien umfaßt (z.B. ein Zuchtmittel, ausgewählt aus Luzernenmahl, Maismahl, Weizenmahl und Molke) oder (b) in Form einer flüssigen Zusammensetzung, die vorzugsweise eine wäßrige Zusammensetzung ist und einen wasserlöslichen milchsäurebakterienspezifischen Nährstoff enthält.

5. Verfahren zum Herstellen einer Zubereitung,
geeignet zur Verwendung bei der Behandlung oder Prophylaxe von Darmkrankheiten bei Tieren, umfassend die Stufen des Impfens eines geeigneten nicht-toxischen organischen Zuchtmittels mit einem Milchsäurebakterien-Impfmittel, umfassend zwei oder mehrere Milchsäurebakterien, die von den Phänotypen folgender Spezies ausgewählt werden, nämlich:
Lactobacillus bavaricus, Lactobacillus casei, ssp. rhamnosus, Lactobacillus coryniformis, Lactobacillus curvatus, Lactobacillus sake, Streptococcus sp. (Enterococcus spp.) und Leuconostoc spp., kultivieren des geimpften Zuchtmittels unter Bedingungen, die die Vervielfachung der Milchsäurebakterien bis zu einer bevorzugten Population ermöglichen und wahlweise Entwässern des Zuchtmittels und seiner zugehörigen Population der Milchsäurebakterien unter Bedingungen, die im wesentlichen nicht-vernichtend für die Milchsäurebaterien sind, um die Zubereitung zu erhalten.

6. Verfahren nach Anspruch 5,
beim dem das Impfmittel eine Mischung von Milchsäurebakterien von zwei oder mehreren (vorzugsweise sämtlichen) Stämme umfaßt:
Lb. sake L2622, Lb. curvatus L2623, Lb. casei (rham) L2624, Lb. curvatus L2625, Lb. sake L2626, Lb. cornyformis L2627, Lb. curvatus L2628, Lb. curvatus L2629, Lb. curvatus L2630, Lb. curvatus L2632, Lb. curvatus L2635, Lb. curvatus L2638, Lb. casei (rham) L2640, Lb. bavaricus L2641, Lb. coryniformis L2643, Lb. sake L2644, Lb. curvatus L2645, Lb. coryniformis L2546, Lb. curvatus L2647, Lb. coryniformis L2651, Lb. coryniformis L2653, Lb. curvatus L2654, Lb. curvatus L2655, Lb. sake L2656, Lb. curvatus L2657, Lb. coryniformis L2658, Lb. curvatus L2659, Streptococcus sp. L2801, Leuconostoc sp. L2806, und Leuconostoc sp. L2807 wobei die Stämme wie im Beispiel 4 (b), einschließlich Tabelle 3, angegeben typisch sind.

7. Verfahren nach Anspruch 6,
bei dem das Impfmittel ferner mindestens eine der Stämme Lb. bavaricus L2648 und Leuconostoc sp. L2803 umfaßt, wie im Beispiel 4 (b), einschließlich Tabelle 3, als typisch angegeben.

**8.** Verfahren nach Anspruch 5, 6 oder 7,
bei dem das Zuchtmittel ausgewählt wird aus Luzernenmahl, Maismahl, Weizenmahl und Molke, vorzugsweise ein Schlamm des Luzernenmahls in Wasser in einem Gewichtverhältnis von etwa 1:3, wobei das Wasser vorzugsweise auf einen pH zwischen 3,5 und 6,9 (z.B. etwa 4,5) eingestellt wird.

**9.** Verfahren nach irgendeinem der Ansprüche 5 - 8,
bei dem das Zuchtmittel ein Fungizid enthält, das nicht-toxisch für Milchsäurebakterien ist, vorzugsweise Natriumsorbat.

**10.** Verfahren nach irgendeinem der Ansprüche 5 - 9,
bei dem das kultivierte Zuchtmittel entwässert wird durch Ausbreiten desselben, über das man dann einen Luftstrom hinwegführt bei einer Temperatur zwischen 15°C und 40°C, wobei das getrocknete Produkt wahlweise durch ein feines Gitter hindurchgeführt und homogenisiert wird, um die Zubereitung in Form eines feinen getrockneten Pulvers zu erhalten.

**11.** Verfahren zum Herstellen eines geeigneten Impfmittels zur Verwendung bei einem Verfahren nach irgendeinem der Ansprüche 5 - 10,
umfassend die Stufen des Sammelns der Inhalte des Verdauungstraktes von geschlachteten, gesunden Tieren, um eine representative Probe solcher Inhalte zu erhalten, Kultivieren der Mikroorganismen in solcher Probe in einem geeigneten Zuchtmittel für Milchsäurebakterien und Entwässern des kultivierten Gemisches, um ein Muttermischungs-Impfmittel von Milchsäurebakterien zu erhalten, wobei das Tier vorzugsweise ein Wiederkäuer, vorzugsweise aus Rindern und Schafen ausgewählt, ist.

**12.** Verfahren zum Herstellen einer flüssigen Zubereitung,
geeignet zur Verwendung bei der Behandlung oder Prophylaxe von Darmkrankheiten bei Tieren, umfassend die Stufen des Vorsehens eines Impfmittels, hergestellt durch ein Verfahren nach irgendeinem der Ansprüche 5 - 10 in einem permeablen Halter dafür (vorzugsweise einer permeablen Papiertüte), Anordnen des Halters in einem flüssigen Zuchtmittel, das Wasser und einen aufgelösten Nährstoff für die Milchsäurebakterien enthält, Ermöglichen, daß die Milchsäurebakterien kultiviert werden und Durchdringen des Halters in das Zuchtmittel hinein und Entfernen des Halters von der so erhaltenen flüssigen Zubereitung, wobei der Nährstoff vorzugsweise eine Kombination der folgenden Stoffe in dem Massenverhältnis enthält, wie es im wesentlichen angegeben ist:

| | |
|---|---|
| Pepton | 4 Teile |
| Hefe Extrakt | 5 Teile |
| Glucose | 8 Teile |
| Triammonium Citrat | 3 Teile |
| Natrium Acetat | 5 Teile |
| Magnesium Sulfat | 0,1 Teile |
| Mangan Sulfat | 0,05 Teile |
| Di-Kalium Phosphat | 2 Teile |
| Kalium Sorbat | 1 Teil |
| Apfelsäure | um den pH der wäßrigen Lösung der Nährstoffe auf etwa 5,8 zu bringen. |

**13.** Verwendung zwei oder mehrerer Milchsäurebakterien, ausgewählt aus den Phänotypen der folgenden Spezies, nämlich:
Lactobacillus bavaricus, Lactobacillus casei ssp. rhamnosus, Lactobacillus coryniformis, Lactobacillus curvatus, Lactobacillus sake, Streptococcus sp. (Enterococcus spp.) und Leuconostoc spp. bei der Herstellung einer pharmazeutischen Zubereitung zur Verwendung bei der Steigerung der Futterumwandlungswirksamkeit bei Tieren oder bei der Behandlung oder Prophylaxe von Darmkrankheiten bei Tieren.

**14.** Kit bzw. Ausrüstung zur Verwendung bei der Herstellung einer flüssigen Zubereitung,
geeignet zur Verwendung in der Behandlung oder Prophylaxe von Darmkrankheiten bei Tieren, umfassend einen Behälter, geeignet mit Wasser gefüllt verwendet zu werden, wobei der Behälter einen wasserlöslichen, trockenen Nährstoff für die Milchsäurebakterien und einen entfernbaren Halter enthält, der für Milchsäurebakterien permeabel ist und ein Bakteriengemisch enthält, wie es in den Ansprüchen 1 - 3 definiert ist, und wobei der Nährstoff vorzugsweise ein Gemisch der folgenden Stoffe enthält, im wesentlichen in dem angegebenen Massenverhältnis:

| Pepton | 4 Teile |
|---|---|
| Hefe Extrakt | 5 Teile |
| Glucose | 8 Teile |
| Triammonium Citrat | 3 Teile |
| Natrium Acetat | 5 Teile |
| Magnesium Sulfat | 0,1 Teile |
| Mangan Sulfat | 0,05 Teile |
| Di-Kalium Sulfat | 2 Teile |
| Kalium Sorbat | 1 Teil |
| Apfelsäure | um den pH des Gemisches des Stoffes in Wasser auf 5,8 zu bringen. |

**15.** Zubereitung nach Anspruch 1,
enthaltend Milchsäurebakterien sämtlicher genannten Spezies.

**Revendications**

**1.** Préparation utilisable dans le traitement ou la prophylaxie de troubles intestinaux chez des animaux, comprenant deux ou plusieurs bactéries lactiques choisies parmi les phénotypes des espèces suivantes, à savoir : *Lactobacillus bavaricus, Lactobacillus casei ssp. rhamnosus, Lactobacillus coryniformis, Lactobacillus curvatus, Lactobacillus sake, Streptococcus sp.* [*Enterococcus spp.*] et *Leuconostoc spp.*

**2.** Préparation selon la revendication 1, qui comprend des bactéries lactiques de deux ou plusieurs (de préférence la totalité) des souches *Lb. sake* L2622, *Lb. curvatus* L2623, *Lb. casei* [*rham*] L2624, *Lb. curvatus* L2625, *Lb. sake* L2626, *Lb. coryniformis* L2627, *Lb. curvatus* L2628, *Lb. curvatus* L2629, *Lb. curvatus* L2630, *Lb. curvatus* L2632, *Lb. curvatus* L2635, *Lb. curvatus* L2638, *Lb. casei* [*rham*] L2640, *Lb. bavaricus* L2641, *Lb. coryniformis* L2643, *Lb. sake* L2644, *Lb. curvatus* L2645, *Lb. coryniformis* L2546, *Lb. curvatus* L2647, *Lb. coryniformis* L2651, *Lb. coryniformis* L2653, *Lb. curvatus* L2654, *Lb. curvatus* L2655, *Lb. sake* L2656, *Lb. curvatus,* L2657, *Lb. coryniformis* L2658, *Lb. curvatus* L2659, *Streptococcus sp.* L2801, *Leuconostoc sp.* L2806 et *Leuconostoc sp.* L2807, les souches étant telles que représentées dans la partie (b) de l'Exemple 4, y compris le Tableau 3.

**3.** Préparation selon la revendication 2, qui comprend de plus une ou plusieurs souches choisies parmi *Lb. bavaricus* L2648 et *Leuconostoc sp.* L2803, telles que représentées dans l'Exemple 4 (b), y compris le Tableau 3.

**4.** Préparation selon l'une quelconque des revendications 1 à 3, qui est (a) sous la forme d'une poudre sèche qui comprend de préférence un support pharmaceutiquement acceptable sous la forme du résidu d'un milieu de croissance approprié pour les bactéries lactiques (par exemple un milieu de croissance choisi parmi la farine de luzerne, la farine de maïs, la farine de blé et le lactosérum) ou (b) sous la forme d'une composition liquide qui est de préférence une composition aqueuse contenant une substance nutritive hydrosoluble spécifique pour les bactéries lactiques.

**5.** Procédé pour produire une préparation utilisable dans le traitement ou la prophylaxie de troubles intestinaux chez des animaux, comprenant les étapes consistant à ensemencer un milieu de croissance organique non toxique approprié avec un inoculum de bactéries lactiques comprenant deux ou plusieurs bactéries lactiques choisies parmi les phénotypes des espèces suivantes, à savoir : *Lactobacillus bavaricus, Lactobacillus casei ssp. rhamnosus, Lactobacillus coryniformis, Lactobacillus curvatus, Lactobacillus sake, Streptococcus sp.* [*Enterococcus spp.*] et *Leuconostoc spp.*, à cultiver le milieu de croissance ensemencé dans des conditions permettant la multiplication des bactéries lactiques jusqu'à une population préférée, et à déshydrater facultativement le milieu et sa population associée de bactéries lactiques dans des conditions qui sont essentiellement non létales

18

pour les bactéries lactiques afin d'obtenir la préparation.

**6.** Procédé selon la revendication 5, dans lequel l'inoculum comprend un mélange de bactéries lactiques consistant en deux ou plusieurs (de préférence la totalité) des souches *Lb. sake* L2622, *Lb. curvatus* L2623, *Lb. casei* [*rham*] L2624, *Lb. curvatus* L2625, *Lb. sake* L2626, *Lb. coryniformis* L2627, *Lb. curvatus* L2628, *Lb. curvatus* L2629, *Lb. curvatus* L2630, *Lb. curvatus* L2632, *Lb. curvatus* L2635, *Lb. curvatus* L2638, *Lb. casei* [*rham*] L2640, *Lb. bavaricus* L2641, *Lb. coryniformis* L2643, *Lb. sake* L2644, *Lb. curvatus* L2645, *Lb. coryniformis* L2546, *Lb. curvatus* L2647, *Lb. coryniformis* L2651, *Lb. coryniformis* L2653, *Lb. curvatus* L2654, *Lb. curvatus* L2655, *Lb. sake* L2656, *Lb. curvatus,* L2657, *Lb. coryniformis* L2658, *Lb. curvatus* L2659, *Streptococcus sp.* L2801, *Leuconostoc sp.* L2806 et *Leuconostoc sp.* L2807, les souches étant telles que représentées dans l'Exemple 4 (b), y compris le Tableau 3.

**7.** Procédé selon la revendication 6, dans lequel l'inoculum comprend également au moins l'une des souches *Lb. bavaricus* L2648 et *Leuconostoc sp.* L2803, telles que représentées dans l'Exemple 4 (b), y compris le Tableau 3.

**8.** Procédé selon la revendication 5, 6 ou 7, dans lequel le milieu de croissance est choisi parmi la farine de luzerne, la farine de maïs, la farine de blé et le lactosérum, de préférence une bouillie de farine de luzerne dans l'eau en un rapport en poids d'environ 1:3, l'eau étant de préférence ajustée à un pH de 3,5 à 6,9 (par exemple d'environ 4,5).

**9.** Procédé selon l'une quelconque des revendications 5 à 8, dans lequel le milieu de croissance comprend un conservateur antimycosique qui est non toxique pour les bactéries lactiques, de préférence le sorbate de potassium.

**10.** Procédé selon l'une quelconque des revendications 5 à 9, dans lequel le milieu de croissance cultivé est déshydraté par étalement du milieu de croissance cultivé et passage d'un courant d'air sur le produit étalé à une température comprise entre 15°C et 40°C, le produit séché étant facultativement passé à travers un tamis fin et homogénéisé pour produire la préparation sous la forme d'une poudre séchée fine.

**11.** Procédé de production d'un inoculum utilisable dans un procédé selon l'une quelconque des revendications 5 à 10, comprenant les étapes consistant à prélever des échantillons du contenu du tube digestif d'un animal sain abattu pour obtenir un échantillon représentatif de ce contenu, à cultiver les microorganismes de cet échantillon dans un milieu de croissance approprié pour les bactéries lactiques et à déshydrater le mélange cultivé pour obtenir un lot maître d'inoculum de bactéries lactiques, l'animal étant de préférence un ruminant, de préférence choisi parmi les bovins et les moutons.

**12.** Procédé pour produire une préparation liquide utilisable dans le traitement ou la prophylaxie de troubles intestinaux chez des animaux, comprenant les étapes consistant à disposer un inoculum produit par un procédé selon l'une quelconque des revendications 5 à 10 dans un réservoir perméable pour celui-ci (de préférence un sac en papier perméable), à placer le réservoir dans un milieu de croissance liquide comprenant de l'eau et une substance nutritive solubilisée pour les bactéries lactiques, à laisser les bactéries lactiques cultiver et passer dans le milieu à travers le réservoir et à retirer le réservoir de la préparation liquide ainsi obtenue, la substance nutritive comprenant de préférence une association des produits suivants dans les proportions en masse sensiblement telles que spécifiées :

| Peptone | 4 parties |
|---|---|
| Extrait de levure | 5 parties |
| Glucose | 8 parties |
| Citrate triammonique | 3 parties |
| Acétate de sodium | 5 parties |
| Sulfate de magnésium | 0,1 partie |
| Sulfate de manganèse | 0,05 partie |
| Phosphate dipotassique | 2 parties |
| Sorbate de potassium | 1 partie |
| Acide malique | pour amener le pH de la solution aqueuse des substances nutritives à environ 5,8. |

13. Utilisation de deux ou plusieurs bactéries lactiques choisies parmi les phénotypes des espèces suivantes, à savoir : *Lactobacillus bavaricus, Lactobacillus casei ssp. rhamnosus, Lactobacillus coryniformis, Lactobacillus curvatus, Lactobacillus sake, Streptococcus sp.* [*Enterococcus spp.*] et *Leuconostoc spp.* dans la fabrication d'une préparation pharmaceutique à utiliser pour accroître le rendement de transformation des aliments chez des animaux ou dans le traitement ou la prophylaxie de troubles intestinaux chez des animaux.

14. Kit à utiliser pour la production d'une préparation liquide utilisable dans le traitement ou la prophylaxie de troubles intestinaux chez des animaux, comprenant un récipient convenant pour être rempli d'eau à l'emploi, le récipient contenant une substance nutritive hydrosoluble sèche pour bactéries lactiques et un réservoir amovible en une matière permable aux bactéries lactiques et contenant un mélange de bactéries tel que défini dans l'une quelconque des revendications 1 à 3, la substance nutritive comprenant de préférence un mélange des produits suivants sensiblement dans les proportions en masse indiquées :

| Peptone | 4 parties |
|---|---|
| Extrait de levure | 5 parties |
| Glucose | 8 parties |
| Citrate triammonique | 3 parties |
| Acétate de sodium | 5 parties |
| Sulfate de magnésium | 0,1 partie |
| Sulfate de manganèse | 0,05 partie |
| Phosphate dipotassique | 2 parties |
| Sorbate de potassium | 1 partie |
| Acide malique | pour amener le pH d'un mélange des produits dans l'eau à 5,8. |

15. Préparation selon la revendication 1, contenant des bactéries lactiques de toutes lesdites espèces.